Europäisches Patentamt

European Patent Office

Office européen des brevets

(11) Publication number: **0 292 601**
**A1**

(12) **EUROPEAN PATENT APPLICATION**

(21) Application number: 87110692.8

(51) Int. Cl.⁴: **A61K 35/84**

(22) Date of filing: 23.07.87

(30) Priority: 14.05.87 JP 117286/87

(43) Date of publication of application:
30.11.88 Bulletin 88/48

(84) Designated Contracting States:
AT BE CH DE ES FR GB GR IT LI LU NL SE

(71) Applicant: **NODA SHOKUKIN KOGYO CO., LTD.**
**121, Shimizu**
**Noda-shi Chiba-ken(JP)**

(72) Inventor: **Iizuka, Chiyokichi**
**c/o Noda Shokukin Kogyo Co., Ltd. 121,**
**Shimizu**
**Noda-shi Chiba-ken(JP)**
Inventor: **Maeda, Hiroaki**
**c/o Noda Shokukin Kogyo Co., Ltd. 121,**
**Shimizu**
**Noda-shi Chiba-ken(JP)**

(74) Representative: **Wey, Hans-Heinrich, Dipl.-Ing.**
**Patentanwälte Wey & Partner**
**Widenmayerstrasse 49**
**D-8000 München 22(DE)**

(54) Anti aids drug extracted from lentinus edodes.

(57) This therapeutic drug, effective for the treatment of AIDS, includes as its effective component a substance extracted from cultured mycelium of *lentinus edodes*
mixture of sugar, protein, and an inorganic element. The sugar may include at least one sugar from the group consisting of glucose, galactose, mannose, xylose, arabinose, fucose, and phamnose; and the inorganic element may include at least one element from the group consisting of potassium, calcium, manganese, iron, nickel, copper, zinc, germanium, bromide, rubidium, and strontium. The mycelium of *lentinus edodes* may desirably be cultured in a solid medium containing as a main component a base substance formed from a true grass plant such as bagasse.

FIG. 1

EP 0 292 601 A1

# ANTI AIDS DRUG EXTRACTED FROM LENTINUS EDODES

## BACKGROUND OF THE INVENTION

The present invention relates to a therapeutic drug which can control the growth of the AIDS virus and which can normalize the immune response of a patient.

The disease AIDS (acquired immune deficiency syndrome) is widely known as a lethal sickness which is characterized by a severe deficiency of the immune system of the patient, and this disease is believed to be caused by an infection by human T-lymphotropic virus type III (HTLV-III), also known as lymphadenopathy-associated virus (LAV), followed by opportunistic infections and generation of tumors. According to the statistics published by the CDC (Center for Disease Control) of the United States, up to the time point of July 20, 1985 there were 11,871 reported cases of AIDS, of which 5,917 resulted in the death of the sufferers. The number of AIDS sufferers is still rapidly increasing. Even in Japan a certain number of AIDS patients and carriers have been discovered, and there is strong public pressure for the taking of immediate action.

The general terminological expression "AIDS" covers all cases of sickness in which adolescent persons suffer from an immune deficiency of an unknown cause and in which the number of T4 helper cells drops sharply with the result that the patient suffers from Kaposi's sarcoma ("SK") and/or from various forms of opportunistic infection.

The body of a living organism such as a human being is maintained to be near a normal state and is protected by various forms of biological mechanism, so that its life is maintained. Such biological mechanisms include maintenance of body temperature near a target level by opening and closing of pores according to the surrounding temperature, regulation of the secretion of digestive enzymes according to the ingestion and digestion of food, metabolism and regeneration of tissue cells for replacing old tissue with new, and immune response. Immune response plays an important part in dealing with pathogenic microorganisms and toxic substances, both of which directly threaten life. In other words, the immune system of the living body recognizes and deals with antigens, and the capability of the immune system to thus handle antigens more or less determines the resistance of the body to diseases. Various types of cells contribute to the response of the immune system, and there are cellular immunity cells (such as T-lymphocytes) which expel and exterminate antigens by directly acting upon such antigens, there are cells which product substances which in their turn activate such cells which are directly responsible for cellular immunity, and there are also humoral immunity cells (such as B-lymphocytes) which produce antibodies, i.e. substances which are capable of rejecting antigens. The activity of all of these cells tends to diminsh as a result of aging, stress, cancers, and infections.

The AIDS virus is capable of destroying the T-Lymphocyte cells, and it is reported that approximately 70% of AIDS patients die from various infections and the like within two years after the appearance of the first symptoms.

The present inventors have made various researches in the last few years related to mycelium of edible mushrooms of the *basidiomycetes* type, and have evolved various processes for extracting medically effective components from cultured mycelium of *lentinus edodes* reports relating to the separation of beta-D-gluconin polysugars having anti-tumor properties from *basidiomycetes* received much attention. For instance, the immunity conferring and activating property of a substance, marketed under the trade mark "LEM" by Noda Shokuhin Kogyo K.K. of Chiba-ken, Japan, which is derived from cultured mycelium of the edible mushroom known as *lentinus edodes* present inventors, and has been claimed, in Japanese Patent Publication Serial No. 53-23392, and successful instances of cure of B-type chronic hepatitis have been reported, for example in the report of the 71st Meeting of the Japanese Society of Gastroenterology, which see Vol. 82, 1105 (1985) of the Journal of the Japanese Society of Gastroenterology. And particularly, in Japanese Patent Application Serial No. 61-115710 (1986), corresponding patent applications to which have been filed in a number of other countries, and which it is not intended hereby to admit as prior art to the present patent application except to the extent in any case mandated by applicable law, the present inventors have described the observation that an immunity activating substance containing sugars having molecular weight in the range of from about 600 to about 1.5 million (A fraction) and 150 to 0.8 million (B fraction) and derived from cultured mycelium is effective, in particular, for curing B-type chronic hepatitis.

## SUMMARY OR THE INVENTION

The inventors of the present invention have expanded the discoveries of the above identified previously applied for patent application, and have discovered that this substance is also effective against the AIDS virus.

Further, it is supposed by the present inventors that similarly effective substances can be extracted from cultured mycelium of *basidiomycetes* other than *lentinus edodes*

Accordingly, it is the primary object of the present invention to provide a therapeutic drug, which is effective for the treatment of AIDS.

It is a further object of the present invention to provide such an AIDS-effective therapeutic drug, which improves the immune response of the patient.

It is a further object of the present invention to provide such an AIDS-effective therapeutic drug, which improves the activity of Interleukin-1.

It is a further object of the present invention to provide such an AIDS-effective therapeutic drug, which has advantageous mitogen effect.

It is a yet further object of the present invention to provide such an AIDS-effective therapeutic drug, which is not toxic.

It is a yet further object of the present invention to provide such an AIDS-effective therapeutic drug, which is not liable to produce undesirable side effects.

According to the most general aspect of the present invention, these and other objects are attained by a therapeutic drug for the treatment of AIDS, comprising as its effective component a substance extracted from cultured mycelium of *lentinus edodes*

mixture of sugar, protein, and an inorganic element. In this case, the sugar may include at least one sugar from the group consisting of glucose, galactose, mannose, xylose, arabinose, fucose, and phamnose; and the inorganic element may include at least one element from the group consisting of potassium, calcium, manganese, iron, nickel, copper, zinc, germanium, bromide, rubidium, and strontium. The mycelium of *lentinus edodes* may desirably be cultured in a solid medium containing as a main component a base substance formed from a true grass plant such as bagasse.

According to such a therapeutic drug as specified above, as will be detailed later, this drug is effective against the AIDS virus. Further, in contrast to conventional anti-viral substances which are typically too toxic and have too severe side effects to be routinely used as treatment for AIDS victims, by contrast this drug according to the present invention, which is 100% naturally derived, as will be shown hereinafter is highly safe and is substantially free of undesirable side effects. Accordingly, it is considered that this drug according to the present invention is an effective drug for treating AIDS patients.


## BRIEF DESCRIPTION OF THE DRAWINGS

The present invention will now be described with respect to the preferred embodiment thereof, and with reference to the illustrative drawings appended hereto, which however are provided for the purposes of explanation and exemplification only, and are not intended to be limitative of the scope of the present invention in any way, since this scope is to be delimited solely by the accompanying claims. Of these figures:

Fig. 1 is a chart showing the effectiveness of LEM-HT, the preferred embodiment of the drug of the present invention, in controlling the appearance of HTLV-III virus; and:

Fig. 2 is a chart showing the effectiveness of LEM-HT, the preferred embodiment of the drug of the present invention, in improving the activity of Interleukin-1.


## DESCRIPTION OF THE PREFERRED EMBODIMENT

The present invention will now be described with reference to the preferred embodiment thereof, and with reference to the figures.

*Method of Preparation of the Preferred Embodiment of the Drug of the Present Invention*

The following method has been evolved for preparation of the preferred embodiment of the drug of the present invention.

A solid medium is prepared, consisting essentially of approximately 90% of bagasse, approximately 5% of rice bran, and approximately 5% of nutritious material (wheat bran or the like may be used).

This thus prepared solid medium is sterilized by a typical method, and then solid seeds of *lentinus edodes* are inoculated into the thus sterilized medium.

Then the thus inoculated medium is cultured for from about four to about six months in a culture chamber, which should be air conditioned to be approximately at room temperature, i.e. at a temperature of from approximately 20° C to approximately 25° C, and which should be maintained to be at a relative humidity of approximately 60%.

Then the medium, in which mycelium should have proliferated, is transferred to a high temperature process chamber in which thermal processing is performed, as follows: the medium is heated up to a temperature of from about 32° C to about 34° C and is maintained at said temperature for from about 24 to about 48 hours. Next the medium is tranferred to a low temperature process chamber in which thermal processing is performed, as follows: the medium is cooled down to a temperature of from about 5° C to about 8° C and is maintained at said temperature, and at a relative relative humidity of approximately 85%, for from about 5 to about 7 days.

This temperature processing is not considered to be absolutely necessary for the production of the preferred embodiment of the drug of the present invention, but is preferred in order to ensure stabilization of the quality of the resultant product. Further, the main material used for the solid medium which is employed as a raw material may be bagasse as described above, but alternatively may also be a product, or a mixture of products, of a true grass or grasses or the like, such as beet residue, rice straw, wheat straw, barley straw, and so on.

After the temperature processing of the medium with the mycelium formed thereon has been completed, said medium is transferred to a culture chamber and is left for some time. Over this time period, the fruiting bodies of *lentinus edodes* start growing out from the surface of the medium. Then, the medium is taken out from the culture chamber and is crushed in a crusher, and the thus crushed medium is dried by heat and air at a temperature of about 80° C for a period of from about three to about four hours, so that its water content is reduced to from about 3% to about 5%, while the mycelium digests itself, so as to promote the metabolism of said mycelium.

Next, to a quantity of about 600 grams of the thus dried medium which contains the products of self digestion and metabolism of the cultured mycelium, there is added about five liters of water, and the mixture is boiled for about one hour, while being stirred. As a result of this stirring, the effective ingredients contained in the products of metabolism and the cellular fluid of the mycelium dissolve into the water.

The suspension fluid thus obtained is filled into a straining bag made of flannel or the like, and then it is strained at a certain degree of pressurization. The strained fluid is then further filtered with a membrane filter which removes any microorgansims which may be present in said fluid, and thus the effective substances contained in the products of the metabolism and in the cellular fluid of the mycelium are extracted. Thereafter, this extracted fluid is pressurized and is condensed by ultrafiltration, and is freeze dried, thus yielding a powder which is brown in color. This powder, which is the preferred embodiment of the therapeutic drug of the present invention, will be termed "LEM-HT" hereinafter in this specification.

*Molecular Composition*

Quantity analyses of the LEM-HT were conducted in the Cellular Biology Class of the Pharmaceutical (Pharmacological) Department of Toyama Medical and Pharmaceutical (Pharmacological) University.

The results of these analyses of this LEM-HT based upon the phenol/condensed sulfuric acid method for detecting sugar and upon the Lowry method for detecting protein were as follows:

**Sugar**     **44.0%**
**Protein**   **24.6%**
**other**     **31.4%**

And the results of these analyses of this LEM-HT for the compositions of the multi-sugars included therein, which are considered as being primarily responsible for the physiological activity thereof, were as follows:

**Glucose**   **27.5%**

**Galactose** 3.1%
**Mannose** 6.7%
**Xylose** 30.9%
**Arabinose** 30.5%
**Fucose** 0.7%
**Phamnose** 0.6%

*Elemental*

Next, the following experiment for determining the elemental composition of this LEM-HT was conducted by Professor Masami Michizaki of the Radiographic Engineering Department of Kobe Mercantile Marine University.

To prepare samples for radiography, about 91.2 mg of LEM-HT was dissolved into about 20 liters of substantially pure water, and Y was added thereto. Three samples, each of approximately 20 microliters, were evaluated in each test. Three radiographic tests were performed using 40 microcuries of radiation exposure provided by 2 MeV protons.

And the results of these analyses of this LEM-HT for the elemental composition thereof were as follows:

**Potassium (K)** 15.09 plus or minus 0.31 mg/g
**Calcium (Ca)** 22.00 plus or minus 0.32 mg/g
**Manganese (Mn)** 1.208 plus or minus 0.027 mg/g
**Iron (Fe)** 2.362 plus or minus 0.047 mg/g
**Nickel (Ni)** 52.5 plus or minus 7.7 mg/g
**Copper (Cu)** 89.1 plus or minus 3.7 mg/g
**Zinc (Zn)** 282.8 plus or minus 11.8 mg/g
**Germanium (Ge)** less than 4 mg/g
**Bromine (Br)** 11.4 plus or minus 3.4 mg/g
**Rubidium (Rb)** 39.4 plus or minus 4.8 mg/g
**Strontium (Sr)** 164.3 plus or minus 8.4 mg/g

Note: The content of each of these elements is expressed as a ratio relative to the dry weight of the sample, and each of the values is an average of the results for the three test samples, while the error in each case is the square root of the sum of the squares of the unbiased variance and the root mean square of the error in the spectral analysis.

*Test of Anti-Aids*

Next, the following experiment for determing the effectiveness of this LEM-HT for inhibiting the appearance of antigen to HTLV-III virus in MT-4 cells which were infected with AIDS virus (HTLV-I positive) was conducted by Professor Naoki Yamamoto of the Parasitology Course of the Medical Department of Yamaguchi University.

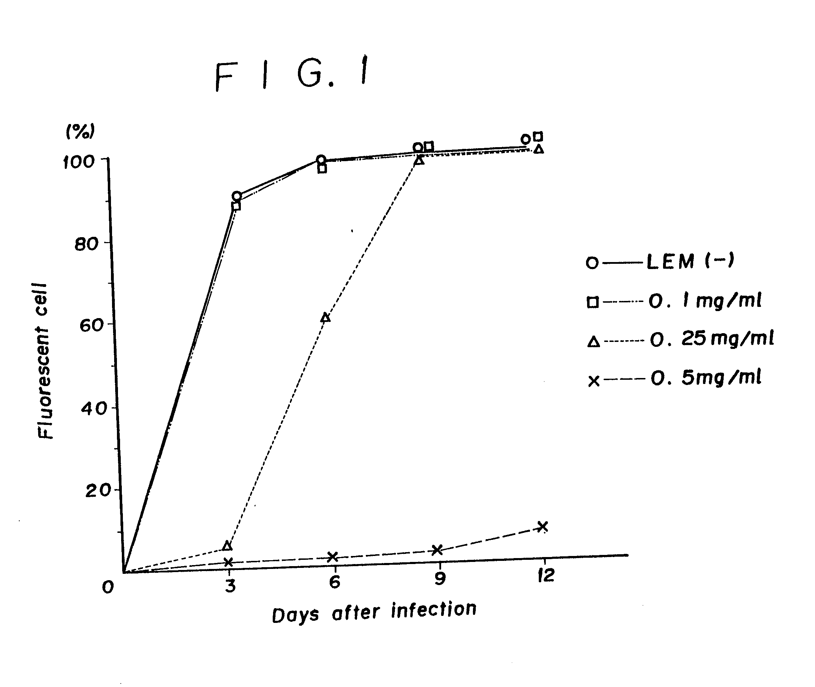

First, MT-4 cells (lymphocytes) which were infected with HTLV-I positive AIDS virus at a concentration of $2 \times 10^5$ cells per milliliter were applied to a 24-hole plate. This was done in four separate trials. The four solutions of LEM-HT were prepared of concentrations respectively substantially 0 mg/ml (pure water), substantially 0.1 mg/ml, substantially 0.25 mg/ml, and substantially 0.5 mg/ml, and each of these four solutions was injected into the holes of one of the plates, thus infecting the HTLV-III with a M.O.I. of 0.001. Thereafter, every third day, the appearance of HTLV-III antigen was measured by the fluorescent antigen method. The results of the experiment are shown in the chart of Fig. 1.

As can be seen from Fig. 1, the LEM-HT was effective in controlling the HTLV-I positive infection of HTLV-III in MT-4 cells. And it is seen that these results were dependent upon the value of the concentration of the LEM-HT, and further it is seen that in fact LEM-HT at a concentration of 0.5 mg/ml can control such an infection.

*Test of Immunity Conferring and Activating*

Next, the following experiment for determining the effectiveness of this LEM-HT for improving the action of Interleukin (IL-1) was conducted by the same person who conducted the test described proximately above.

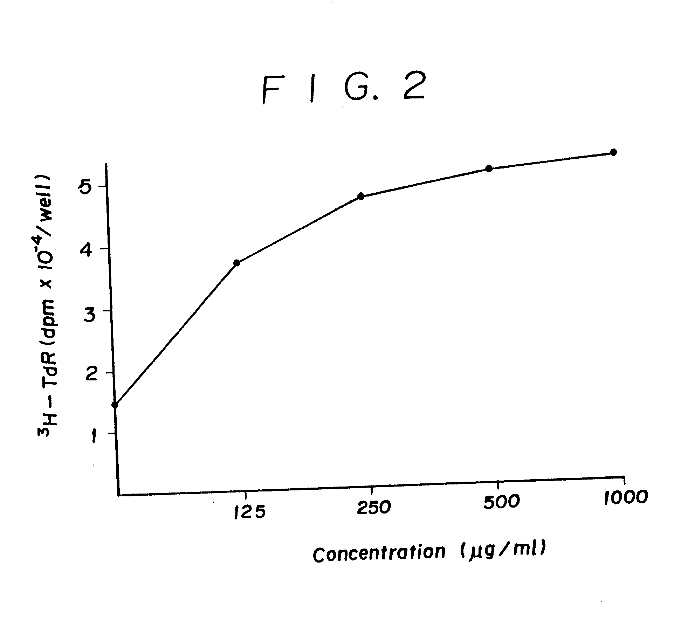

Liquidus paraffin was injected into the abdominal cavity of a guinea pig, and the macrophages exuding therefrom were collected and the concentration thereof was adjusted to approximately $5 \times 10^6$ cells per milliliter. Then LEM-HT was added, in five experimental cases in which concentration of respectively approximately zero mg/ml (substantially none), approximately 125 mg/ml, approximately 250 mg/ml, approximately 500 mg/ml, and approximately 1000 mg/ml, to this fluid containing these macrophage cells suspended therein, and in each case the resultant solution was cultured at a temperature of about 37° C for a period of about 48 hours. Then in each case the IL-1 contained in the clear part of this cultured microphage fluid thus processed with LEM-HT was measured by a thymocyte proliferation assay. Further, in each case, this clear part of this cultured microphage fluid thus processed with LEM-HT was then added to C3H/He J mouse thymus cells, and this solution was then further cultured for a period of about 48 hours. Then, in each case, a quantity of about 1 micro Ci/ml of 3H-thymidine was added to the cultured solution, and after this solution was then cultured for a further period of about 30 hours the take out of the 3H-thymidine into the acid insoluble fraction was measured with a liquid scintillation counter. The results of the experiment are shown in the chart of Fig. 2.

As can be seen from Fig. 2, the LEM-HT, when its concentration was 125 mg/ml or more, was effective in stimulating the microphage cells and in improving the activity of Interleukin-1. This shows that the capability of production of T-lymphocytes was qaulitatively improved, and further that the capability of producing antibodies was improved.

*Test of Mitogen Effect*

Next, the following experiment for determining the mitogen effect of this LEM-HT on mouse spleen cells was conducted.

Rat spleen was scissored into pieces, was squeezed in a saline solution, and was filtered through a stainless steel strainer of 80 mesh.

The filtrate was then centrifuged, and the resulting pellet was suspended in 0.35% NaCl for hemolysis.

The suspension was then centrifuged, and the resulting splenic cells were washed with Hanks solution which contained kanamycin to the concentration of about 60 micrograms/ml.

The washed cells were then suspended, at a concentration of about $10^7$ cells per milliliter, in a TC199 medium which contained about 20% of FBS and which also contained kanamycin to the concentration of about 60 micrograms/ml.

An aliquot of this suspension liquid (concentration of about $10^6$ cells per milliliter) was then incubated in the presence of LAP1 in an environment containing $CO_2$ to a portion of about 5% at a temperature of about 37° C for a period of about 24 hours.

Next, a concentration of about 0.1 micro Ci of 3H-thymidine was added to the liquid, and it was incubated for a further 24 hours.

The cells were collected onto a glass fiber membrane sheet, and were then in succession washed with saline solution (three times), with a 5% solution of TCA (three times), with 95% ethanol (twice), and with diethylether (once).

Finally, the radiation of the flocculated cells was measured with a scintillation counter.

The results are shown in the following Table:

| Mouse | micrograms/culture | | | | | |
|-------|------|------|------|------|------|------|
| No. | 0.4 | 1 | 2 | 4 | 10 | 20 |
| 1 | 1.39 | 1.33 | 1.51 | 1.08 | 2.46 | 2.25 |
| 2 | 0.98 | 1.00 | 1.18 | 1.51 | 2.13 | 2.01 |
| 3 | 1.01 | 1.20 | 1.39 | 1.46 | 2.02 | 2.10 |
| 4 | 1.46 | 1.37 | 1.68 | 1.82 | 2.35 | 2.49 |
| 5 | 1.16 | 1.37 | 1.38 | 1.63 | 2.23 | 2.00 |
| 6 | 1.27 | 1.56 | 1.38 | 1.75 | 2.41 | 2.38 |
| Mean | 1.21 | 1.31 | 1.42 | 1.69 | 2.27 | 2.21 |
| $\pm \sigma_{n-1}$ | 0.20 | 0.19 | 0.17 | 0.20 | 0.17 | 0.20 |

where $\sigma_{n-1} = \sqrt{\Sigma (x_i - x)^2 / (n-1)}$

As can be seen from these above detailed experimental results, LEM-HT at the concentration of 0.4 micrograms/culture or more promoted the rejuvenation of mouse spleen cells and displayed a clear mitogen effect.

Thus, it is seen as described above that the preferred embodiment of the drug of the present invention, i.e. LEM-HT, can produce a direct effect for controlling the growth of the AIDS virus, and that such LEM-HT can directly act upon a living organism so as to strengthen immunity and so as to produce a therapeutic effect. In other words, it is estimated that LEM-HT is highly effective against the AIDS virus, because LEM-HT can control the reduction of T-lymphocytes due to infection by such AIDS virus via its action to stimulate macrophages, to increase the IL-1 activity, and to promote the production of antibodies.

*Acute Toxic Test*

Next, the acute toxic properties of this drug LEM-HT were tested by administering it orally, subcutaneously, and abdominally to SD rats.

The results obtained for LD 50 values (95% confidence level) were as follows (units gm/kg):

| | male | female |
|-------|------|--------|
| Oral | 17.2 | 15.8 |
| Subcutaneous | 4.5 | 5.2 |
| Abdominal | 3.3 | 3.1 |

The deaths in the cases of oral and of subcutaneous intake occurred within one or two days irrespective of the gender of the test animals, but there was a difference between the male rats and the female rats for the time interval before death in the case of abdominal intake: the male rats died within two days, while the female rats died within three days. When massive dosages were given, the body parts involved in such dosages suffered acute inflammation; in other words, in the case of oral dosages the digestive organs suffered bleeding, in the case of subcutaneous dosages the thighs suffered from necrosis, and in the case of abdominal dosages adhesion took place between the livers and the diaphragms of the test animals.

According to the general observations which were made regarding the deaths of the test animals, these deaths were caused by loss of body strength due to incapacity to cope with the sudden changes which resulted from the administration of the drug.

According to this acute toxic test, it was determined that LEM-HT is as safe as normal foodstuffs. Therefore, the present inventors conducted tests of this drug on a few AIDS patients.

*Clinical Tests on AIDS Patients*

These tests were performed by Dr. M. Moslek and Dr. Kevin Lance Jones at the Emperor's College of Traditional Oriental Medicine in the USA.

Case 1: Male, aged 46

This person was tested because of complaints of a high fever and a sense of severe fatigue, and was diagnosed as an AIDS sufferer because HTLV-III antibodies were detected. His T4-cell count was 1,250 per mm³. LEM-HT was administered orally at the rate of 6 gm/day. As a result, the T4-cell count increased to 2,045 after 30 days and to 2,542 after 60 days, and he started to feel better.

Case 2: Male, aged 34

This patient suffered from Kaposi's sarcoma due to infection by the AIDS virus, and his T4-cell count was 822 per mm³. LEM-HT was administered orally at the rate of 9 gm/day. As a result, the T4-cell count increased to 1,050 and he started to get better, but eventually died as a result of contracting pneumonia.

Case 3: Male, aged 43

This patient had no symptoms, but he was found to be infected with HTLV-III; thus, he was a so-called carrier. LEM-HT was administered orally at the rate of 6 gm/day. As a result, after 60 days, the virus antigen disappeared.

*Test Summary*

As a result of these tests, the two doctors involved reported that LEM-HT, which is 100% naturally derived, appeared to be safe and not to cause any adverse side effects in the patients.

*Conclusion*

As can be seen from the results of these various tests, the LEM-HT which is the preferred embodiment of the drug of the present invention is effective against the AIDS virus. It should be noted that, although convention anti-viral substances are typically too toxic and have too severe side effects to be routinely used as treatment for AIDS victims, by contrast LEM-HT, which is 100% naturally derived, is highly safe and is free of side effects. Accordingly, it is considered that this drug according to the present invention is an effective drug for treating AIDS patients.

Although the present invention has been shown and described in terms of the preferred embodiment thereof, and with reference to the appended drawings, it should not be considered as being particularly limited thereby, since the details of any particular embodiment, or of the drawings, could be varied without, in many cases, departing from the ambit of the present invention. Accordingly, the scope of the present invention is to be considered as being delimited, not by any particular perhaps entirely fortuitous details of the disclosed preferred embodiment, or of the drawings, but solely by the scope of the accompanying claims, which follow.

**Claims**

1. A therapeutic drug for the treatment of AIDS, comprising as its effective component a substance extracted from cultured mycelium of *lentinus edodes*

2. A therapeutic drug according to claim 1, wherein a main component of said effective component is a mixture of sugar, protein, and an inorganic element.

3. A therapeutic drug according to claim 2, wherein said sugar includes at least one sugar from the group consisting of glucose, galactose, mannose, xylose, arabinose, fucose, and phamnose.

4. A therapeutic drug according to claim 2, wherein said inorganic element includes at least one element from the group consisting of potassium, calcium, manganese, iron, nickel, copper, zinc, germanium, bromide, rubidium, and strontium.

5. A therapeutic drug according to claim 1, wherein said mycelium of *lentinus edodes* is cultured in a solid medium containing as a main component a base substance formed from a true grass plant.

F I G. 1

F I G. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 154 066 (NODA SHOKKIN KOGYO CO., LTD) * Claims 1,2,6; page 2, line 15 - page 3, line 4 * | 1,5 | A 61 K 35/84 |
| | --- | | |
| A | EP-A-0 088 151 (NODA SHOKUKIN KOGYO CO., LTD) * Claims 1-5; page 4, line 1 - page 6, line 4 * | 1 | |
| | --- | | |
| X | CHEMICAL ABSTRACTS, vol. 106, 2nd March 1987, page 29, abstract no. 60908r, Columbus, Ohio, US; H. SUZUKI et al.: "Immunological activities of high-molecular-weight fractions purified from lentinus edodes mycelia extract (LEM)-macrophage-activating effect and mitogenic activity" & IGAKU NO AYUMI 1986, 138(6/7), 441-2 * Abstract * | 1 | |
| | ----- | | TECHNICAL FIELDS SEARCHED (Int. Cl.4) |
| | | | A 61 K |

The present search report has been drawn up for all claims

| Place of search | Date of completion of the search | Examiner |
|---|---|---|
| THE HAGUE | 13-09-1988 | COUCKE A.O.M. |

EPO FORM 1503 03.82 (P0401)